# EUROPEAN PATENT APPLICATION

(11) **EP 4 767 931 A1**
(43) Date of publication of application: **01.07.2026**
(21) Application number: 24223453.2
(22) Date of filing: 27.12.2024
(51) Int. Cl.: A61B 5/145

(54) **ANALYTE SENSOR BASED ON BLOOD GLUCOSE MONITORING**

(71) Applicant: Syai UK Ltd, Cambridge CB2 8DL (GB)
(72) Inventor: WANG, Zhihua, Cambridge, CB2 8DL (GB)
(74) Representative: Nederlandsch Octrooibureau

(57) **Abstract**

The present application provides an analyte sensor based on blood glucose monitoring. The analyte sensor comprises a substrate, a working electrode, a reference electrode, a counter electrode, and a sensing layer; the sensing layer is provided on the working electrode; the working electrode, reference electrode, and counter electrode are provided at surfaces of the substrate, and the two electrodes from the working electrode; the working electrode, reference electrode, and counter electrode located at the same surface of the substrate are spatially separated by a channel. The analyte sensor can be implanted into a human body for monitoring, and has the characteristics of good flexibility, small size, low manufacturing cost, simple process flow, and long-term monitoring.

## Description

### Technical Field

The present application belongs to the technical field of sensors, and specifically relates to an analyte sensor based on blood glucose monitoring.

### Background

Flexible electrodes refer to electrodes manufactured using flexible substrates. Biosensors made of flexible electrodes have the characteristics of light weight, high flexibility, stretchability, and excellent comfort. Biosensors made of flexible electrodes are widely used in testing of nucleic acids, proteins, glucose, bacteria, toxins, cells, and pesticides in vitro.

At present, the vast majority of flexible electrodes are used for temporary in vitro testing. With the increasing demand, there is a growing need for sensors that can continuously monitor analytes in vivo. However, the current size of flexible electrodes is relatively large, which cannot meet the adaptability of the human body to sensors. At the same time, the complex manufacturing process flow and high manufacturing costs of sensors further limit the development of analyte sensors.

### Summary

In order to improve the shortcomings of the prior art, the present application provides an analyte sensor based on blood glucose monitoring. The analyte sensor can be implanted into a human body to monitor the analyte. The analyte sensor has the characteristics of good flexibility, small size, low manufacturing cost, simple process flow, and long-term monitoring.

The purpose of the present application is achieved through the following technical solution:
An analyte sensor, characterized in that, the analyte sensor comprises a substrate, a working electrode, a reference electrode, a counter electrode, and a sensing layer; the sensing layer is provided on the working electrode; the working electrode, reference electrode, and counter electrode are provided at surfaces of the substrate, and the two electrodes located at the same surface of the substrate are spatially separated by a channel;
the width of the channel is 1µm-280µm; the length of the channel is 30µm-10,000µm.

According to the present application, the analyte sensor is, for example, a sensor for analyzing blood glucose, such as a blood glucose sensor.

The beneficial effects of the present invention are as follows:
The present application provides an analyte sensor based on blood glucose monitoring. The analyte sensor includes a channel structure, and the analyte sensor can be implanted into a human body for monitoring, and has the characteristics of good flexibility, small size, low manufacturing cost, simple process flow, and long-term monitoring. The introduction of the channel structure of the present application makes the thickness of the analyte sensor more suitable and the wearing experience better. The introduction of the channel structure can also avoid problems such as unstable signal transmission caused by small electrode area or narrow electrode cables, resulting in poor sensor stability.

### Brief Description of the Drawings

FIG. 1 is a view of the sensor electrode structure A in Embodiment 1.
FIG. 2 is a structural diagram of the section A-A' of the sensor electrode structure A in Embodiment 1.
FIG. 3 is a structural diagram of the section B-B' of the sensor electrode structure A in Embodiment 1.
FIG. 4 is a structural diagram of the section C-C' of the sensor electrode structure A in Embodiment 1.
FIG. 5 is a trend chart of blood glucose changes after 14 days of implantation of sensor electrode structure A into a human body in Embodiment 1.
FIG. 6 is a view of the sensor electrode structure B in Embodiment 2.
FIG. 7 is a structural diagram of the section A-A' of the sensor electrode structure B in Embodiment 2.
FIG. 8 is a structural diagram of the section B-B' of the sensor electrode structure B in Embodiment 2.
FIG. 9 is a structural diagram of the section C-C' of the sensor electrode structure B in Embodiment 2.
FIG. 10 is a view of the sensor electrode structure C in Embodiment 3.
FIG. 11 is a structural diagram of the section A-A' of the sensor electrode structure C in Embodiment 3.
FIG. 12 is a structural diagram of the section B-B' of the sensor electrode structure C in Embodiment 3.
FIG. 13 is a structural diagram of the section C-C' of the sensor electrode structure C in Embodiment 3.
FIG. 14 is a view of the sensor electrode structure D in Embodiment 4.
FIG. 15 is a structural diagram of the section A-A' of the sensor electrode structure D in Embodiment 4.
FIG. 16 is a structural diagram of the section B-B' of the sensor electrode structure D in Embodiment 4.
FIG. 17 is a structural diagram of the section C-C' of the sensor electrode structure D in Embodiment 4.
FIG. 18 is a view of the sensor electrode structure E in Embodiment 5.
FIG. 19 is a structural diagram of the section A-A' of the sensor electrode structure E in Embodiment 5.
FIG. 20 is a structural diagram of the section B-B' of the sensor electrode structure E in Embodiment 5.
FIG. 21 is a structural diagram of the section C-C' of the sensor electrode structure E in Embodiment 5.
FIG. 22 is a view of the sensor electrode structure F in Embodiment 6.
FIG. 23 is a structural diagram of the section A-A' of the sensor electrode structure F in Embodiment 6.
FIG. 24 is a structural diagram of the section B-B' of the sensor electrode structure F in Embodiment 6.
FIG. 25 is a structural diagram of the section C-C' of the sensor electrode structure F in Embodiment 6.
FIG. 26 is a view of the sensor electrode structure G in Embodiment 7.
FIG. 27 is a structural diagram of the section A-A' of the sensor electrode structure G in Embodiment 7.
FIG. 28 is a structural diagram of the section B-B' of the sensor electrode structure G in Embodiment 7.
FIG. 29 is a structural diagram of the section C-C' of the sensor electrode structure G in Embodiment 7.
FIG. 30 is a view of the sensor electrode structure H in Embodiment 8.
FIG. 31 is a structural diagram of the section A-A' of the sensor electrode structure H in Embodiment 8.
FIG. 32 is a structural diagram of the section B-B' of the sensor electrode structure H in Embodiment 8.
FIG. 33 is a structural diagram of the section C-C' of the sensor electrode structure H in Embodiment 8.
FIG. 34 is a partial view of the protrusion position of the sensor of the present application.
FIG. 35 is a schematic diagram of the combination of the sensor with a protruding structure and the auxiliary device (see left), as well as a schematic diagram of the combination of the sensor without a protruding structure and the auxiliary device (see right), where the reference number 1000 is the auxiliary device, and 1001 is the sensor.
FIG. 36 is a structural diagram of the sensor surface after implantation of the sensor with the protruding structure into the human body (see left), as well as a structural diagram of the sensor surface after implantation of the sensor without the protruding structure into the human body (see right).
FIG. 37 is a structural diagram of the sensor after implantation of the sensor with the channel structure into the human body (see left), as well as a structural diagram of the sensor after implantation of the sensor without the channel structure (i.e., stacked structure) into the human body (see right).
FIG. 38 is an enlarged partial view of the sensor according to the preferred solution of the present application.
FIG. 39 is a change trend chart of blood glucose monitoring using the working electrode of the sensor electrode structure implanted into the human body for one day in Comparative Example 1.
FIG. 40 is a change trend chart of blood glucose monitoring using the working electrode of the sensor electrode structure implanted into the human body for two days in Comparative Example 2.

### Detailed Description of the Preferred Embodiments

### <analyte sensor>

As mentioned earlier, the present application provides an analyte sensor, characterized in that, the analyte sensor comprises a substrate 100, a working electrode, a reference electrode, a counter electrode, and a sensing layer 115; the sensing layer 115 is provided on the working electrode; the working electrode, reference electrode, and counter electrode are provided at surfaces of the substrate 100, and the two electrodes from the working electrode; the working electrode, reference electrode, and counter electrode located at the same surface of the substrate 100 are spatially separated by a channel 114.

According to the embodiments of the present application, the working electrode, reference electrode, and counter electrode are provided at surfaces of the substrate 100, that is, any two of the working electrode, reference electrode, and counter electrode are located at the same surface of the substrate 100; such a structure results in two different functional areas/electrode areas on the first surface of the sensor, which are spatially separated by a channel 114 and do not affect each other. This allows the two functional areas/electrode areas to function on the same side surface of the sensor, while there is one functional area/electrode area on a second surface of the sensor opposite to the first surface. Such a structure can ensure that the functional area/electrode area of the sensor has sufficient area, thereby enabling the sensor to have good stability during long-term monitoring; in addition, the structures of the two functional areas/electrode areas located on the same side surface of the sensor can also make the manufacturing process flow of the sensor relatively simple, while ensuring that the obtained sensor has good flexibility, smaller size, and lower cost; moreover, due to the provision of the reference electrode, the analyte sensor can achieve long-term monitoring of the analyte after implantation into the body.

Research has found that if the working electrode, reference electrode, and counter electrode are provided on the same side surface of the substrate, and due to the limited implantation width of the sensor itself, the area of each electrode itself and each electrode cable will be narrow, which can easily cause problems such as short circuits and open circuits. If the working electrode, reference electrode, and counter electrode are designed in a stacked structure (i.e., the spatial positions of two electrodes are in a vertical relationship, separated by a dielectric layer in the middle, and there is no spatial channel structure), this structure increases the manufacturing process flow and difficulty, and the thickness is larger, which will affect the wearing experience and reduce the stability of the sensor. In the present application, two functional areas are provided at the same side of the substrate, and spatially separated by a channel, which ensures sufficient electrode area and avoids problems such as electrode short circuits and open circuits; at the same time, due to the moderate thickness of the electrode, the wearing experience of the analyte sensor will be better and the stability will be higher.

According to the embodiments of the present application, the working electrode, reference electrode, and counter electrode are provided at surfaces of the substrate 100, forming a structure in which two electrodes are provided on the first surface of the substrate 100, and one electrode is provided on the second surface of the substrate 100 opposite to the first surface. The two electrodes refer to any two of the working electrode, reference electrode, and counter electrode; the mentioned one electrode refers to the remaining electrode other than the two mentioned above.

According to the embodiments of the present application, the working electrode, reference electrode, and counter electrode are provided surfaces of the substrate 100, for example, the working electrode and the reference electrode are provided on a first surface of the substrate, spatially separated by a channel, and the counter electrode is provided on a second surface of the substrate opposite to the first surface; alternatively, the working electrode and the counter electrode are provided on a first surface of the substrate, spatially separated by a channel, and the reference electrode is provided on a second surface of the substrate opposite to the first surface; alternatively, the counter electrode and the reference electrode are provided on a first surface of the substrate, spatially separated by a channel, and the working electrode is provided on a second surface of the substrate opposite to the first surface.

According to the embodiments of the present application, the two electrodes located at the same surface of the substrate are on the same plane, and specifically, the electrode layers, electrode cables and electrode contacts of the two electrodes are on the same plane.

According to the embodiments of the present application, the two electrodes located on the same side of the substrate are partially on the same plane. For example, the electrode layers of the two electrodes are not on the same plane, but the electrode cables and electrode contacts of the two electrodes are on the same plane; alternatively, the electrode cables of the two electrodes are not on the same plane, but the electrode layers and electrode contacts of the two electrodes are on the same plane; alternatively the electrode contacts of the two electrodes are not on the same plane, but the electrode cables and electrode layers of the two electrodes are on the same plane; alternatively the electrode layers and electrode contacts of the two electrodes are not on the same plane, but the electrode cables of the two electrodes are on the same plane; alternatively the electrode cables and electrode contacts of the two electrodes are not on the same plane, but the electrode layers of the two electrodes are on the same plane; alternatively the electrode cables and electrode layers of the two electrodes are not on the same plane, but the electrode contacts of the two electrodes are on the same plane.

According to the embodiments of the present application, the working electrode, reference electrode, and counter electrode are not arranged in a stacked manner, that is, the working electrode, reference electrode, and counter electrode are not stacked on each other. Even if the two electrodes located on the same side of the substrate are on the same plane, they will not likewise be stacked on each other, but will be spatially separated; more precisely, the two electrodes located on the same side of the substrate are on the same plane and spatially separated by a channel; alternatively, the two electrodes located at the same surface of the substrate are partially on the same plane and spatially separated by a channel.

### <structure and composition of the substrate>

According to the embodiments of the present application, the substrate 100 comprises a front end 101, a middle end 102, and a distal end 103, which are sequentially connected.

According to the embodiments of the present application, the analyte sensor 100 has an L-shape, which includes a first side and a second side, and an angle of 45°-135° is formed at the connection between the first side and the second side, preferably 88°-98°, such as 90°, 92°, 93°, 94°, 95°, 96°, 97° or 98°.

According to the embodiments of the present application, the front end 101 is located at the first side and at the non-connected end of the first side, the distal end 103 is located at the second side and at the non-connected end of the second side, and the middle end 102 is located at the first side and second side and at the connected end of the first side and second side. The connected end refers to the end close to the connection between the first side and the second side, while the non-connected end refers to the end far away from the connection between the first side and the second side.

According to the embodiments of the present application, the material forming the substrate comprises at least one of plastic (such as polyethylene terephthalate PET, polyimide PI, polyvinyl chloride PVC, or polyethylene PE), polydimethylsiloxane PDMS, ceramics, glass, paper, and textiles.

According to the embodiments of the present application, the length of the second side of the L-shaped substrate is 1mm-40mm, the length of the first side of the L-shaped substrate is 0.05mm-20mm, and the thickness of the substrate is 20µm-5000µm; preferably, the length of the second side of the L-shaped substrate is 2mm-30mm, the length of the first side of the L-shaped substrate is 0.10mm-10mm, and the thickness of the substrate is 50µm-2000µm; for example, the length of the second side of the L-shaped substrate is 3mm, 4mm, 5mm, 8mm, 10mm, 12mm, 15mm, 18mm, 20mm, 25mm, 28mm, or 30mm; the length of the first side of the L-shaped substrate is 0.10mm, 0.5mm, 1mm, 2mm, 3mm, 4mm, 5mm, 6mm, 8mm, or 10mm.

### <structure and composition of the working electrode>

According to the embodiments of the present application, the working electrode comprises a working electrode layer 107, a working electrode cable 108, and a working electrode contact 109, and the working electrode layer 107 is connected to the working electrode contact 109 through the working electrode cable 108.

For example, the working electrode layer 107 is located at the front end 101 of the substrate, the working electrode cable108 is located at the middle end 102 of the substrate, and the working electrode contact 109 is located at the distal end 103 of the substrate; alternatively, the working electrode layer 107 and the working electrode cable 108 are located at the middle end 102 of the substrate, and the working electrode contact 109 is located at the distal end 103 of the substrate.

According to the embodiments of the present application, a sensing layer 115 is provided on the working electrode layer, and the working electrode mainly functions to react with blood glucose.

According to the embodiments of the present application, the material forming the working electrode comprises one or more of carbon, gold, silver, copper, and their composite materials; alternatively, the material forming the working electrode comprises one or more of precious metals, transition metals, transition metal oxides, and transition metal hydroxides with catalytic/signal conversion properties. For example, the precious metals include gold and its composite materials, platinum and its composite materials; the transition metals include copper and its composite materials, nickel and its composite materials, cobalt and its composite materials, zinc and its composite materials, manganese and its composite materials, etc.

According to the embodiments of the present application, the working electrode is modified onto the substrate through process methods including but not limited to printing, inkjet printing, electrohydrodynamic (EHD) printing, dispensing, spraying, chemical vapor deposition (CVD), physical vapor deposition (PVD), magnetron sputtering, molecular beam epitaxy, electrochemical deposition, electroplating, and evaporation deposition.

According to the embodiments of the present application, the thickness of the working electrode layer is 1µm-20µm, preferably 5µm-15µm, such as 2µm, 3µm, 4µm, 5µm, 6µm, 7µm, 8µm, 9µm, 10µm, 11µm, 12µm, 15µm or 18µm.

### <structure and composition of the reference electrode>

According to the embodiments of the present application, the reference electrode comprises a reference electrode layer 113, a reference electrode cable 110, and a reference electrode contact 112. The reference electrode layer 113 is provided on the reference electrode cable 110, and the reference electrode cable 110 is connected to the reference electrode contact 112.

For example, the reference electrode layer 113 is located at the front end 101 of the substrate, the reference electrode cable 110 is located at the front end 101 and middle end 102 of the substrate, and the reference electrode contact 112 is located at the distal end 103 of the substrate; alternatively, the reference electrode layer 113 and the reference electrode cable 110 are located at the middle end 102 of the substrate, and the reference electrode contact 112 is located at the distal end 103 of the substrate.

According to the embodiments of the present application, the reference electrode is configured to form a voltage circuit with the working electrode, providing a guarantee for converting blood glucose signals into electrochemical signals.

According to the embodiments of the present application, the material forming the reference electrode comprises Ag and AgCl, where the mass ratio of Ag to AgCl is 1:10-10:1, preferably 2:8-8:2, such as 3:7, 4:6, 5:5, 6:4, 7:3.

According to the embodiments of the present application, the reference electrode is modified onto the substrate through process methods including but not limited to printing, inkjet printing, electrohydrodynamic (EHD) printing, dispensing, spraying, chemical vapor deposition (CVD), physical vapor deposition (PVD), magnetron sputtering, molecular beam epitaxy, electrochemical deposition, electroplating, and evaporation deposition.

According to the embodiments of the present application, the thickness of the reference electrode layer is 0.1µm-500µm, preferably 1µm-200µm, such as 5µm, 10µm, 15µm, 20µm, 50µm, 80µm, 90µm, 100µm, 120µm, 150µm, 180µm or 200µm.

### <structure and composition of the counter electrode>

According to the embodiments of the present application, the counter electrode comprises a counter electrode layer 104, a counter electrode cable 105, and a counter electrode contact 106. The counter electrode layer 104 is connected to the counter electrode contact 106 through the counter electrode cable 105.

For example, the counter electrode layer 104 is located at the front end 101 of the substrate, the counter electrode cable 105 is located at the middle end 102 of the substrate, and the counter electrode contact 106 is located at the distal end 103 of the substrate; alternatively, the counter electrode layer 104 and the counter electrode cable 105 are located at the middle end 102 of the substrate, and the counter electrode contact 106 is located at the distal end 103 of the substrate.

According to the embodiments of the present application, the counter electrode is to configured form a current closed circuit with the working electrode, providing a guarantee for converting blood glucose signals into electrochemical signals.

According to the embodiments of the present application, the material forming the counter electrode comprises one or more of carbon, gold, silver, copper, and their composite materials.

According to the embodiments of the present application, the counter electrode is modified onto the substrate through process methods including but not limited to printing, inkjet printing, electrohydrodynamic (EHD) printing, dispensing, spraying, chemical vapor deposition (CVD), physical vapor deposition (PVD), magnetron sputtering, molecular beam epitaxy, electrochemical deposition, electroplating, and evaporation deposition.

According to the embodiments of the present application, the thickness of the counter electrode layer is 1µm-20µm, preferably 5µm-15µm, such as 2µm, 3µm, 4µm, 5µm, 6µm, 7µm, 8µm, 9µm, 10µm, 11µm, 12µm, 15µm or 18µm.

### <sensing layer>

According to the embodiments of the present application, the sensing layer 115 is provided on the working electrode layer 107 of the working electrode.

According to the embodiments of the present application, the material forming the sensing layer 115 comprises one or more of carbon, gold, silver, copper, and their composite materials; alternatively, the material forming the working electrode comprises one or more of glucose oxidase, glucose dehydrogenase, precious metals, transition metals, transition metal oxides, and transition metal hydroxides. For example, the precious metals include gold and its composite materials, platinum and its composite materials; the transition metals include copper and its composite materials, nickel and its composite materials, cobalt and its composite materials, osmium and its composite materials, zinc and its composite materials, manganese and its composite materials, iron and its composite materials.

According to the embodiments of the present application, the sensing layer is formed on the working electrode through process methods including but not limited to adsorption, printing, inkjet printing, electrohydrodynamic (EHD) printing, dispensing, spraying, chemical vapor deposition (CVD), physical vapor deposition (PVD), magnetron sputtering, molecular beam epitaxy, electrochemical deposition, and evaporation deposition.

According to the embodiments of the present application, the thickness of the sensing layer is 1µm-20µm, preferably 1µm-10µm, such as 1µm, 2µm, 3µm, 4µm, 5µm, 6µm, 7µm, 8µm, 9µm, 10µm, 11µm, 12µm, 15µm or 18µm.

According to the embodiments of the present application, the sensing layer is configured to monitor analyte, which is blood glucose.

### <provision of the electrode>

As far as the present application, the working electrode, counter electrode and reference electrode are spatially separated by a channel, preferably the electrode layer of one electrode and the electrode cable of the other electrode located at the same side of the substrate are spatially separated by a channel.

According to the embodiments of the present application, the electrodes located at the same surface of the substrate are, for example, a working electrode and a reference electrode, and the working electrode layer 107 of the working electrode is located at the front end 101 of the substrate; the reference electrode layer 113 of the reference electrode is located at the middle end 102 of the substrate, and the reference electrode and the working electrode are spatially separated by a channel; alternatively, the working electrode layer 107 of the working electrode is located at the middle end 102 of the substrate, the reference electrode layer 113 of the reference electrode is located at the front end 101 of the substrate, and the reference electrode and the working electrode are spatially separated by a channel.

According to the embodiments of the present application, the electrodes located at the same surface of the substrate are, for example, a working electrode and a counter electrode, and the working electrode layer 107 of the working electrode is located at the front end 101 of the substrate; the counter electrode layer 104 of the counter electrode is located at the middle end 102 of the substrate, and the counter electrode and the working electrode are spatially separated by a channel; alternatively, the working electrode layer 107 of the working electrode is located at the middle end 102 of the substrate, the counter electrode layer 104 of the counter electrode is located at the front end 101 of the substrate, and the counter electrode and the working electrode are spatially separated by a channel.

According to the embodiments of the present application, the electrodes located at the same surface of the substrate are, for example, a counter electrode and a reference electrode, and the reference electrode layer 113 of the reference electrode is located at the front end 101 of the substrate; the counter electrode layer 104 of the counter electrode is located at the middle end 102 of the substrate, and the reference electrode and the counter electrode are spatially separated by a channel; alternatively, the reference electrode layer 113 of the reference electrode is located at the middle end 102 of the substrate, the counter electrode layer 104 of the counter electrode is located at the front end 101 of the substrate, and the reference electrode and the counter electrode are spatially separated by a channel.

According to the embodiments of the present application, when the electrodes located at the same surface of the substrate are a working electrode and a reference electrode, the working electrode and the reference electrode are spatially separated by a channel; for example, when the working electrode layer 107 of the working electrode is located at the front end 101 of the substrate, and the reference electrode layer 113 of the reference electrode is located at the middle end 102 of the substrate, the reference electrode layer 113 of the reference electrode is separated from the working electrode cable 108 of the working electrode by a channel; when the working electrode layer 107 of the working electrode is located at the middle end 102 of the substrate, and the reference electrode layer 113 of the reference electrode is located at the front end 101 of the substrate, the reference electrode cable 110 of the reference electrode is spatially separated from the working electrode layer 107 of the working electrode by a channel.

According to the embodiments of the present application, when the electrodes located at the same surface of the substrate are a working electrode and a counter electrode, the working electrode and the counter electrode are spatially separated by a channel; for example, when the working electrode layer 107 of the working electrode is located at the front end 101 of the substrate, and the counter electrode layer 104 of the counter electrode is located at the middle end 102 of the substrate, the counter electrode layer 104 of the counter electrode is separated from the working electrode cable 108 of the working electrode by a channel; when the working electrode layer 107 of the working electrode is located at the middle end 102 of the substrate, and the counter electrode layer 104 of the counter electrode is located at the front end 101 of the substrate, the counter electrode cable 105 of the counter electrode is spatially separated from the working electrode layer 107 of the working electrode by a channel.

According to the embodiments of the present application, when the electrodes located at the same surface of the substrate are a counter electrode and a reference electrode, the counter electrode and the reference electrode are spatially separated by a channel; for example, when the reference electrode layer 113 of the reference electrode is located at the front end 101 of the substrate, and the counter electrode layer 104 of the counter electrode is located at the middle end 102 of the substrate, the counter electrode layer 104 of the counter electrode is separated from the reference electrode cable 110 of the reference electrode by a channel; when the reference electrode layer 113 of the reference electrode is located at the middle end 102 of the substrate, and the counter electrode layer 104 of the counter electrode is located at the front end 101 of the substrate, the counter electrode cable 105 of the counter electrode is spatially separated from the reference electrode layer 113 of the reference electrode by a channel.

According to the embodiments of the present application, when the electrodes at the same side of the substrate are a working electrode and a reference electrode, the working electrode and the reference electrode are on the same plane. Specifically, the working electrode layer 107 of the working electrode, the working electrode cable 108 of the working electrode, the working electrode contact 109 of the working electrode, the reference electrode layer 113 of the reference electrode, the reference electrode cable 110 of the reference electrode, and the reference electrode contact 112 of the reference electrode are all on the same plane; alternatively, the working electrode and the reference electrode are on the same plane. Specifically, at least one component of the electrode layers, electrode cables and electrode contacts of the working electrode and the reference electrode are not on the same plane; for example, the electrode layers of the working electrode and the reference electrode are not on the same plane, the electrode cables of the working electrode and the reference electrode are on the same plane, and the electrode contacts of the working electrode and the reference electrode are on the same plane.

According to the embodiments of the present application, when the electrodes located at the same surface of the substrate are a working electrode and a counter electrode, the working electrode and the counter electrode are on the same plane. Specifically, the working electrode layer 107 of the working electrode, the working electrode cable 108 of the working electrode, the working electrode contact 109 of the working electrode, the counter electrode layer 104 of the counter electrode, the counter electrode cable 105 of the counter electrode, and the counter electrode contact 106 of the counter electrode are all on the same plane; alternatively, the working electrode and the counter electrode are located on the same plane. Specifically, at least one component of the electrode layers, electrode cables, and electrode contacts of the working electrode and the counter electrode is not on the same plane; for example, the electrode layer of the working electrode and the electrode layer of the counter electrode are not on the same plane, the electrode cables of the working electrode and the electrode cables of the counter electrode are on the same plane, and the electrode contacts of the working electrode and the counter electrode are on the same plane.

According to the embodiments of the present application, when the electrodes located at the same surface of the substrate are a counter electrode and a reference electrode, the counter electrode and the reference electrode are on the same plane. Specifically, the counter electrode layer 104 of the counter electrode, the counter electrode cable 105 of the counter electrode, the counter electrode contact 106 of the counter electrode, the reference electrode layer 113 of the reference electrode, the reference electrode cable 110 of the reference electrode, and the reference electrode contact 112 of the reference electrode are all on the same plane; alternatively, the counter electrode and the reference electrode are partially on the same plane. Specifically, at least one component of the electrode layers, electrode cables, and electrode contacts of the counter electrode and reference electrode are not on the same plane; for example, the electrode layers of the counter electrode and the reference electrode are not on the same plane, the electrode cables of the counter electrode and the reference electrode are on the same plane, and the electrode contacts of the counter electrode and the reference electrode are on the same plane.

### <structure of the channel>

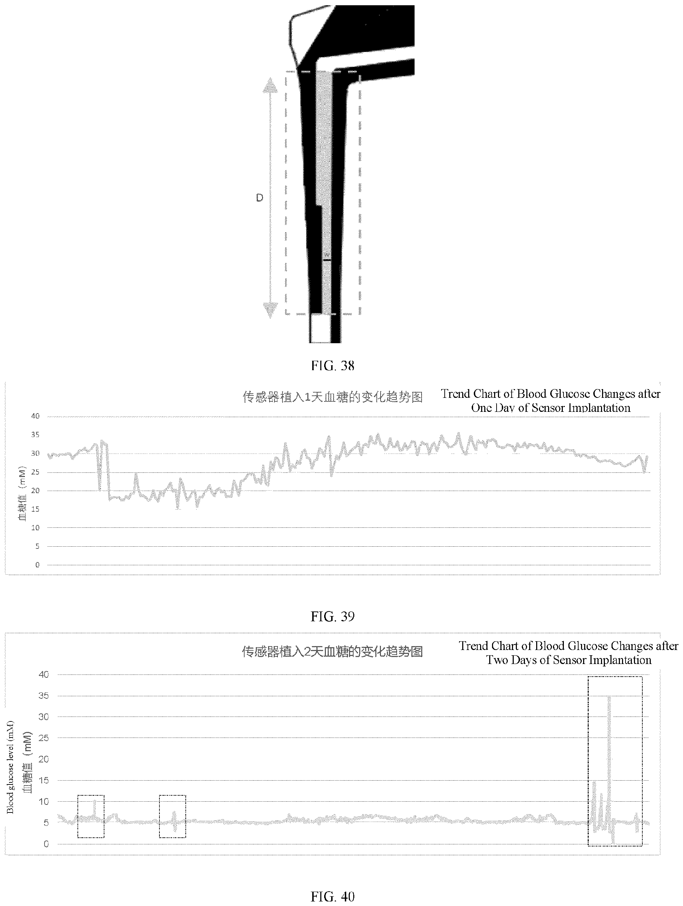

According to the embodiments of the present application, as shown in FIG. 38, the width of the channel 114 refers to the distance between the electrode layer of one electrode and the electrode cable of the other electrode spatially separated by a channel; the line segment "W" in FIG. 38 represents the width of the channel 114; the specific electrode layers and electrode cables need to be determined according to different arrangements of the working electrode, reference electrode, and counter electrode inside the sensor; for example, the width of the channel 114 refers to the distance between the counter electrode cable 105 of the counter electrode and the reference electrode layer 113 of the reference electrode; or the distance between the electrode cable 105 and the working electrode layer 107 of the working electrode; or the distance between the working electrode cable 108 of the working electrode and the counter electrode layer 104 of the counter electrode; or the distance between the working electrode cable 108 of the working electrode and the reference electrode layer 113 of the reference electrode; or the distance between the reference electrode cable 110 of the reference electrode and the working electrode layer 107 of the working electrode; or the distance between the reference electrode cable 110 of the reference electrode and the counter electrode layer 104 of the counter electrode.

According to the embodiments of the present application, the width of the channel 114 may be constant or gradually changing; if it is constant, the width of the channel is constant within the range of 1µm-280µm; if it is gradually changing, the width of the channel 114 gradually increases or decreases along the direction from the front end 101 of the substrate to the middle end 102 of the substrate, and the width of the channel varies within a width range of 1µm-280µm, for example, within a width range of 50µm-150µm or within a width range of 80µm-200µm.

According to the embodiments of the present application, the width of the channel is 1µm-280µm, preferably 50µm-170µm, such as 20µm, 30µm, 40µm, 50µm, 60µm, 70µm, 80µm, 90µm, 100µm, 110µm, 120µm, 130µm, 140µm, 150µm, 160µm, 170µm, 180µm, 190µm, 200µm, 210µm, 220µm, 230µm, 240µm, 250µm, 260µm, 270µm, 280µm, or a range consisting of the two endpoint values mentioned above. Research has found that when the width of the channel is 1µm-280µm, it can effectively separate the two electrodes located on the same side surface of the sensor, enabling the functions of two functional areas/electrode areas on the same side surface of the sensor and improving the stability of the sensor; when the width of the channel is less than 10µm, due to the narrow width of the channel, there is a risk of contact between the two electrodes, resulting in a short circuit between the electrodes and causing the test data to be too high or too low, and resulting in sensor failure; when the width of the channel is greater than 280µm, due to the wide channel width, the electrode/electrode cable may be too narrow, which may result in insufficient functional area/electrode area, or the electrode may be disconnected due to the narrow electrode/electrode cable, leading to data "jumping" or "jittering" during the sensor testing process, affecting the stability of the test data and causing sensor failure.

According to the embodiments of the present application, as shown in FIG. 38, the length of the channel 114 refers to the distance from the front end of the electrode layer located at the middle end of the substrate to the connection between the first side and the second side of the substrate, that is, the line segment "D" in FIG. 38 represents the length of the channel 114; the specific electrode layer needs to be determined according to different arrangements of the working electrode, reference electrode, and counter electrode inside the sensor; for example, the length of the channel 114 is the distance from the front end of the working electrode layer of the working electrode located at the middle end of the substrate to the connection between the first side and the second side of the substrate; or the distance from the front end of the reference electrode layer of the reference electrode located at the middle end of the substrate to the connection between the first and second sides of the substrate; or the distance from the front end of the counter electrode layer of the counter electrode located at the middle end of the substrate to the connection between the first side and the second side of the substrate.

According to the embodiments of the present application, the length of the channel is 30µm-10000µm, such as 30µm, 40µm, 50µm, 60µm, 70µm, 80µm, 90µm, 100µm, 110µm, 120µm, 130µm, 140µm, 150µm, 160µm, 170µm, 180µm, 190µm, 200µm, 210µm, 220µm, 230µm, 240µm, 250µm, 260µm,270µm, 280µm, 300µm, 320µm, 350µm, 380µm, 400µm, 450µm, 500µm, 550µm, 600µm, 700µm, 800µm, 900µm, 1,000µm, 1,100µm, 1200µm, 1,300µm, 1,400µm, 1,500µm, 2,000µm, 2,500µm, 3,000µm, 3,500µm, 4,000µm, 4,500µm, 5,000µm, 5,500µm, 6,000µm, 6,500µm, 7,000µm, 7,500µm, 8,000µm, 8,500µm, 9,000µm, 9,500µm or 10,000µm. Research has found that, when the length of the channel is 30µm-10,000µm, it can effectively monitor the monitored object and provide good wearing comfort; when the length of the channel is less than 30µm, there is a risk that the analyte sensor cannot effectively contact the body fluid, thereby leading to the inability to monitor; when the length of the channel is greater than 10,000µm, due to excessive implantation, the wearer is prone to bleeding and pain, which affects the wearing experience.

According to the embodiments of the present application, the channel can be obtained through printing, engraving, etching, inkjet printing, electrohydrodynamic (EHD) printing, dispensing, spraying, chemical vapor deposition (CVD), physical vapor deposition (PVD), magnetron sputtering, molecular beam epitaxy, electrochemical deposition, and evaporation deposition and other methods.

### <structure of the sensor>

According to the embodiments of the present application, the analyte sensor is a three-electrode system, and the combination of three electrodes can stabilize the potential.

According to the embodiments of the present application, the analyte sensor can continuously monitor for a period of 7-360 days.

According to the embodiments of the present application, the analyte sensor has an L-shape. On the one hand, the L-shaped analyte sensor can be adapted to the auxiliary device to better complete analysis work; on the other hand, the L-shape can effectively prevent sensor damage during implantation, increasing the probability of successful sensor implantation.

According to the embodiments of the present application, the analyte sensor has an L-shape, which includes a first side and a second side, and an angle of 45°-135° is formed at the connection between the first side and the second side, preferably 88°-98°.

According to the embodiments of the present application, there are two contact areas and two electrode areas on a first surface of the analyte sensor, and there is one contact area and one electrode area on a second surface of the analyte sensor opposite to the first surface. Furthermore, the electrode area is located at the first side of the L-shaped analyte sensor, and the contact area is located at the second side of the sensor.

### <protruding structure>

According to the embodiments of the present application, a protruding structure 118 is formed at the connection between the first side and the second side. Preferably, the outer edge of the connection between the first side and the second side forms a protruding structure, and the provision of the protruding structure can adapt the sensor to the auxiliary device during implantation, and can make the sensor more smooth during implantation.

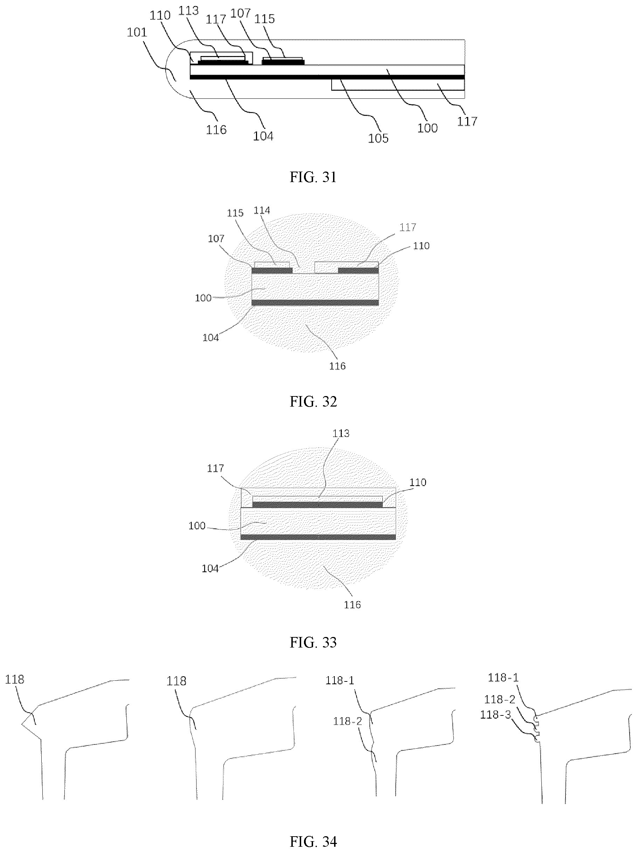

According to the embodiments of the present application, the protruding structure can be semicircular, triangular, square, or polygonal, as shown in FIG. 34.

According to the embodiments of the present application, there may be one protruding structure, and may also be more protruding structures, as shown in FIG. 34.

### <dielectric layer>

According to the embodiments of the present application, the analyte sensor further comprises a dielectric layer 117, which is provided on the substrate, electrode or electrode cable and can be adjusted according to the structure of the sensor, and the dielectric layer is configured to provide insulation to protect the working electrode, counter electrode and reference electrode from damage.

For example, the dielectric layer is provided on the surface of the working electrode or working electrode cable to protect the working electrode from damage; the dielectric layer is provided on the surface of the reference electrode or reference electrode cable to protect the reference electrode from damage; the dielectric layer is provided on the surface of the counter electrode or counter electrode cable to protect the counter electrode from damage.

According to the embodiments of the present application, the material forming the dielectric layer comprises at least one of plastic (such as polyethylene terephthalate PET, polyimide PI, polyvinyl chloride PVC, or polyethylene PE), polydimethylsiloxane PDMS, ceramics, glass, paper, and textiles.

According to the embodiments of the present application, the dielectric layer is formed on the reference electrode, working electrode cable or counter electrode cable through process methods including but not limited to adsorption, printing, inkjet printing, electrohydrodynamic (EHD) printing, dispensing, spraying, chemical vapor deposition (CVD), physical vapor deposition (PVD), magnetron sputtering, molecular beam epitaxy, electrochemical deposition, and evaporation deposition.

According to the embodiments of the present application, the thickness of the dielectric layer is 1µm-1,000µm, preferably 10µm-500µm, such as 10µm, 20µm, 50µm, 80µm, 100µm, 120µm, 150µm, 180µm, 200µm, 220µm, 250µm, 260µm,280µm, 300µm, 320µm, 350µm, 380µm, 400µm, 420µm, 450µm, 480µm, 500µm, 600µm, 700µm, 800µm, 900µm or 1,000µm.

In all sensor configurations disclosed herein, various electrodes may be separated from each other by channels.

### <protective layer>

According to the embodiments of the present application, the analyte sensor further comprises a protective layer 116, which is provided on the outermost layer of the sensor.

According to the embodiments of the present application, the protective layer may be a single-layer structure or a multi-layer structure.

According to the embodiments of the present application, the protective layer may be configured to limit flux. The protective layer may be configured to prevent biofouling. The protective layer may be configured to shield environmental noise. The protective layer may be configured to protect internal components/elements from corrosion and damage.

According to the embodiments of the present application, the protective layer can be in the form of gel or solid.

According to the embodiments of the present application, the protective layer may be a component, such as a filter.

According to the embodiments of the present application, the material forming the protective layer may be ethylene homopolymer or ethylene copolymer.

According to the embodiments of the present application, the thickness of the protective layer is 0.5µm-2000µm, preferably, the thickness of the protective layer is 2µm-1000µm, such as 5µm, 10µm, 50µm, 80µm, 100µm, 150µm, 200µm, 250µm, 300µm, 350µm, 400µm, 450µm, 500µm, 600µm, 700µm, 800µm, 900µm or 1,000µm.

According to the embodiments of the present application, the protective layer is modified onto the sensor through processes including but not limited to dispensing, spin coating, spray coating, dip coating, blade coating, nesting, wrapping, printing, inkjet printing, electrohydrodynamic (EHD) printing, electrospinning, dispensing, spraying, chemical vapor deposition (CVD), physical vapor deposition (PVD), magnetron sputtering, molecular beam epitaxy, electrochemical deposition, and evaporation deposition.

### <instructions for sensors>

According to the embodiments of the present application, the monitoring technology of the analyte sensor for blood glucose can be any one of the first generation, second generation, third generation, or fourth generation. Specifically, different testing technologies is configured to monitor blood glucose based on the different working electrode materials and sensing layer materials.

According to the embodiments of the present application, the analyte sensor may contain enzymes.

According to the embodiments of the present application, the analyte sensor comprises a working electrode, a reference electrode, and a counter electrode. The analyte sensor is implanted into the body through minimally invasive means using an auxiliary device. Specifically, the front end and partial middle end of the analyte sensor (i.e., the position below the protruding structure, one side of the L-shape) are configured to be implanted into the body. The working electrode and the counter electrode are configured to form a current circuit, and the working electrode and the reference electrode are configured to form a voltage circuit, wherein the sensing layer located on the working electrode is configured to react with blood glucose to generate an electrochemical signal; furthermore, there is a corresponding relationship between the intensity of the generated electrochemical signal and the blood glucose concentration, that is, the higher the blood glucose concentration, the stronger the generated electrochemical signal.

According to the embodiments of the present application, an implant length of the analyte sensor is configured to be 1mm-20mm, and the width is configured to be 0.05mm-10mm; preferably, an implant length of the analyte sensor is configured to be 3mm-18mm, and the width is configured to be 0.10mm-8mm.

### <testing method for determining analytes>

A testing method for determining analytes, comprising:
1) an analyte sensor is exposed to a fluid containing different concentrations of analytes, and voltage is applied to the sensor, in order to obtain electrochemical signals of different signal intensities; the signal intensity of the electrochemical signal is proportional to the concentration of the analyte in the fluid;
2) the signal intensity of electrochemical signals is associated with the concentration of analytes in the fluid, and a standard curve of the signal intensity of electrochemical signals and the concentration of analytes is drawn;
3) the analyte sensor is exposed to a fluid containing the monitored analyte, and voltage is applied to the sensor, in order to obtain an electrochemical signal, thus the concentration of the monitored analyte in the fluid is obtained based on the signal intensity of the obtained electrochemical signal.

According to the embodiments of the present application, the analyte is blood glucose.

The present application will be further clarified below in combination with the specific embodiments. It should be understood that the following embodiments are only illustrative and explanatory of the present application, and should not be interpreted as limiting the scope of protection of the present application. All technologies implemented based on the above content of the present application are covered within the scope of protection intended by the present application.

Unless otherwise specified, experimental methods applied in the following embodiments are conventional methods; unless otherwise specified, all the materials and reagents applied in the following embodiments are commercially available.

### Embodiment 1

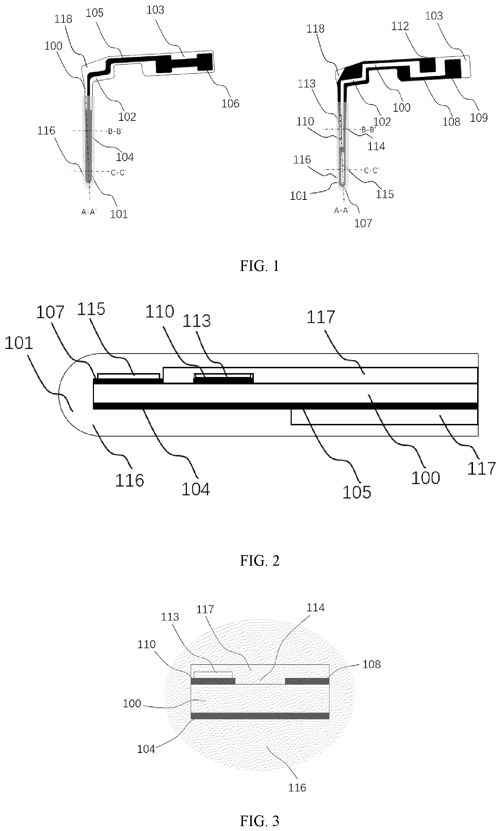

The structure of the sensor electrode structure A in Embodiment 1 is as shown in FIGs. 1-4.

FIG. 1 is a view of the sensor electrode structure A in Embodiment 1, where the left figure is a back view of the sensor electrode structure A and the right figure is a front view of the sensor electrode structure A. From FIG. 1, it can be seen that the sensor electrode structure A comprises a substrate 100: the substrate 100 has an L-shaped structure, and the substrate 100 comprises a front end 101, a middle end 102, and a distal end 103;
The sensor electrode structure A comprises a working electrode, which comprises a working electrode layer 107 located at the front end 101 of the substrate, a working electrode cable 108 located at the middle end 102 of the substrate, and a working electrode contact 109 located at the distal end 103 of the substrate; the sensor electrode structure A comprises a sensing layer 115 located on the working electrode layer 107; the sensor electrode structure A comprises a reference electrode, which comprises a reference electrode layer 113 and a reference electrode cable 110 located at the middle end 102 of the substrate, a reference electrode contact 112 located at the distal end 103 of the substrate, and the reference electrode layer 113 is provided on the reference electrode cable 110; the sensor electrode structure A comprises a counter electrode, which comprises a counter electrode layer 104 located at the front end 101 of the substrate, a counter electrode cable 105 located at the middle end 102 of the substrate, and a counter electrode contact 106 located at the distal end 103 of the substrate; the counter electrode is located on the back of the sensor electrode structure A, and the reference electrode and the working electrode are located on the front of the sensor electrode structure A.

The sensor electrode structure A comprises a channel 114 located between the working electrode cable 108 of the working electrode and the reference electrode layer 113, and the working electrode and the reference electrode are separated by the channel 114; the width of the channel 114 is adjustable within the range of 1µm-280µm, and the length of the channel is adjustable within the range of 30µm-10,000µm.

The sensor electrode structure A has an L-shape, which includes a first side and a second side, and an angle of 85°-95° is formed at the connection between the first side and the second side. A protruding structure 118 is formed at the outer edge of the connection between the first side and the second side.

The sensor electrode structure A comprises a protective layer 116, which is provided outside of the sensor.

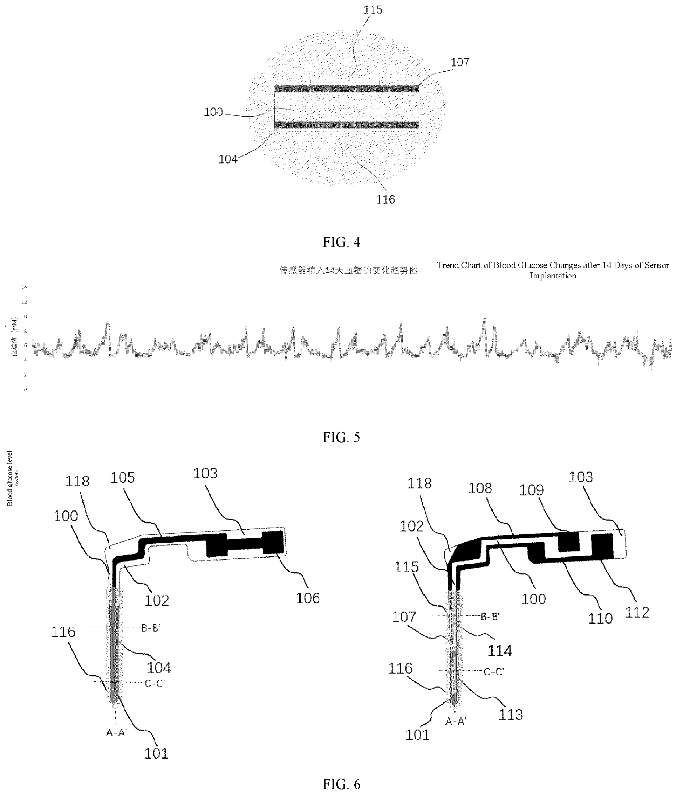

FIG. 2 is a structural diagram of the section A-A' of the sensor electrode structure A in Embodiment 1. FIG. 3 is a structural diagram of the section B-B' of the sensor electrode structure A in Embodiment 1. FIG. 4 is a structural diagram of the section C-C' of the sensor electrode structure A in Embodiment 1. It can be seen from FIGs. 2-4 that, the sensor electrode structure A also comprises a dielectric layer 117, which is provided on the reference electrode, working electrode cable, and counter electrode cable.

The above-mentioned sensor electrode structure A is implanted into the human body for long-term testing. FIG. 5 is a change trend chart of blood glucose after 14 days of implantation. It can be seen from FIG. 5 that the sensor can achieve long-term monitoring of blood glucose.

### Embodiment 2

The structure of the sensor electrode structure B in Embodiment 2 is as shown in FIGs. 6-9.

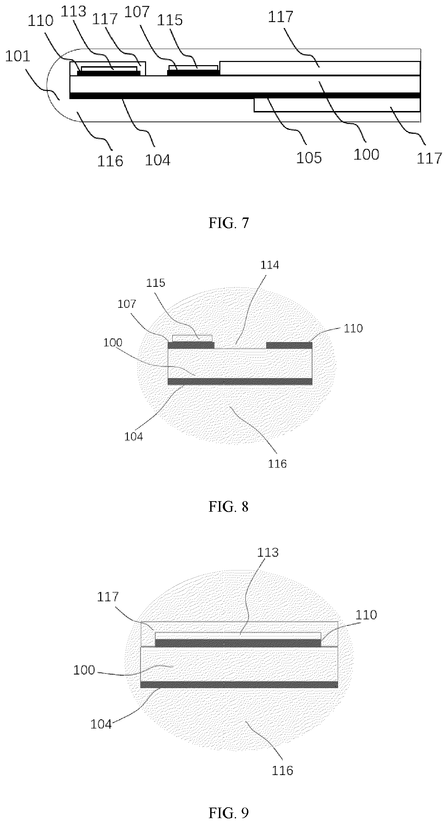

FIG. 6 is a view of the sensor electrode structure B in Embodiment 2, where the left figure is a back view of the sensor electrode structure B and the right figure is a front view of the sensor electrode structure B. FIG. 7 is a structural diagram of the section A-A' of the sensor electrode structure B in Embodiment 2. FIG. 8 is a structural diagram of the section B-B' of the sensor electrode structure B in Embodiment 2. FIG. 9 is a structural diagram of the section C-C' of the sensor electrode structure B in Embodiment 2.

The composition of the sensor electrode structure B is exactly the same as that of the sensor electrode structure A, except that the position relationship between the reference electrode and the working electrode located on the front of the sensor electrode structure has changed, that is, the sensor electrode structure B comprises a working electrode, which comprises a working electrode layer 107 and a working electrode cable 108 located at the middle end 102 of the substrate, and a working electrode contact 109 located at the distal end 103 of the substrate;
The sensor electrode structure B comprises a reference electrode, which comprises a reference electrode layer 113 located at the front end 101 of the substrate, a reference electrode cable 110 located at the front end 101 and the middle end 102 of the substrate, and a reference electrode contact 112 located at the distal end 103 of the substrate; the reference electrode layer 113 is provided on the reference electrode cable 110;
The sensor electrode structure B comprises a channel 114 located between the working electrode layer 107 of the working electrode and the reference electrode cable 110 of the reference electrode, and the working electrode and the reference electrode are separated by the channel 114; the width of the channel 114 is adjustable within the range of 1µm-280µm, and the length of the channel is adjustable within the range of 30µm-10,000µm.

### Embodiment 3

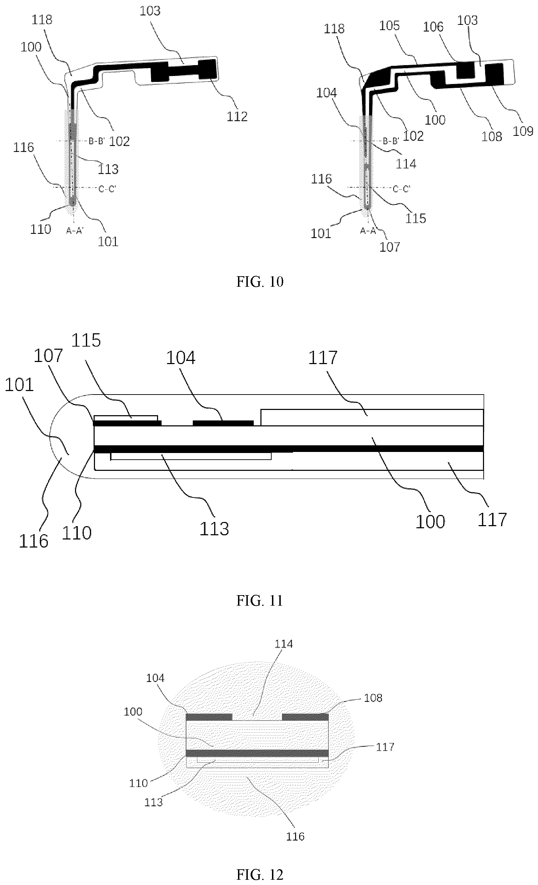

The structure of the sensor electrode structure C in Embodiment 3 is as shown in FIGs. 10-13.

FIG. 10 is a view of the sensor electrode structure C in Embodiment 3, where the left figure is a back view of the sensor electrode structure C and the right figure is a front view of the sensor electrode structure C. From FIG. 10, it can be seen that the sensor electrode structure C comprises a substrate 100: the substrate 100 has an L-shaped structure, and the substrate 100 comprises a front end 101, a middle end 102, and a distal end 103;
The sensor electrode structure C comprises a working electrode, which comprises a working electrode layer 107 located at the front end 101 of the substrate, a working electrode cable 108 located at the middle end 102 of the substrate, and a working electrode contact 109 located at the distal end 103 of the substrate;
The sensor electrode structure C comprises a sensing layer 115 located on the working electrode layer 107;
The sensor electrode structure C comprises a reference electrode, which comprises a reference electrode layer 113 located at the front end 101 of the substrate, a reference electrode cable 110 located at the front end 101 and the middle end 102 of the substrate, and a reference electrode contact 112 located at the distal end 103 of the substrate; the reference electrode layer 113 is provided on the reference electrode cable 110;
The sensor electrode structure C comprises a counter electrode, which comprises a counter electrode layer 104 located at the middle end 102 of the substrate, a counter electrode cable 105 located at the middle end 102 of the substrate, and a counter electrode contact 106 located at the distal end 103 of the substrate;
The reference electrode is located on the back of the sensor electrode structure C, and the counter electrode and the working electrode are located on the front of the sensor electrode structure C.

The sensor electrode structure C comprises a channel 114 located between the working electrode cable 108 of the working electrode and the counter electrode layer 104, and the working electrode and the counter electrode are separated by the channel 114; the width of the channel 114 is adjustable within the range of 1µm-280µm, and the length of the channel is adjustable within the range of 30µm-10,000µm.

The sensor electrode structure C has an L-shape, which includes a first side and a second side, and an angle of 85°-95° is formed at the connection between the first side and the second side. A protruding structure 118 is formed at the outer edge of the connection between the first side and the second side.

The sensor electrode structure C comprises a protective layer 116, which is provided outside of the sensor.

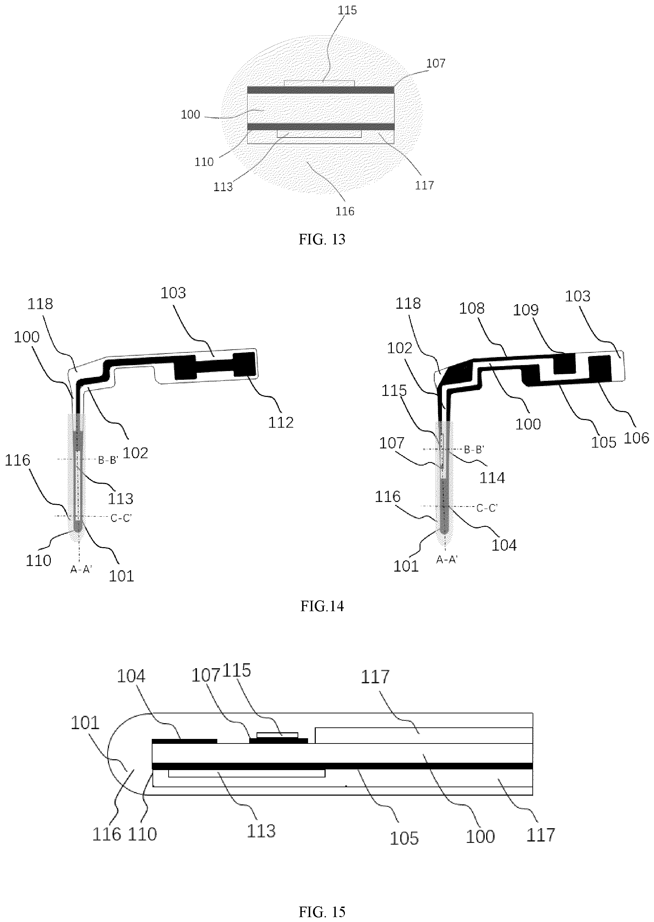

FIG. 11 is a structural diagram of the section A-A' of the sensor electrode structure C in Embodiment 3. FIG. 12 is a structural diagram of the section B-B' of the sensor electrode structure C in Embodiment 3. FIG. 13 is a structural diagram of the section A-A' of the sensor electrode structure C in Embodiment 3. From FIGs. 11-13, it can be seen that that the sensor electrode structure C also comprises a dielectric layer 117, which is provided on the reference electrode, working electrode cable, and counter electrode cable.

### Embodiment 4

The structure of the sensor electrode structure D in Embodiment 4 is as shown in FIGs. 14-17.

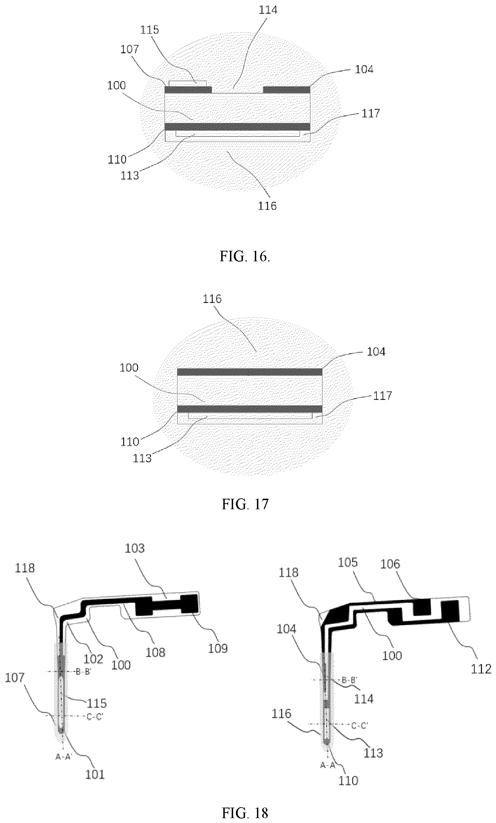

FIG. 14 is a view of the sensor electrode structure D in Embodiment 4, where the left figure is a back view of the sensor electrode structure D and the right figure is a front view of the sensor electrode structure D. FIG. 15 is a structural diagram of the section A-A' of the sensor electrode structure D in Embodiment 4. FIG. 16 is a structural diagram of the section B-B' of the sensor electrode structure D in Embodiment 4. FIG. 17 is a structural diagram of the section C-C' of the sensor electrode structure D in Embodiment 4.

The composition of the sensor electrode structure D is exactly the same as that of the sensor electrode structure C, except that the position relationship between the counter electrode and the working electrode located on the front of the sensor electrode structure has changed, that is, the sensor electrode structure D comprises a working electrode, which comprises a working electrode layer 107 and a working electrode cable 108 located at the middle end 102 of the substrate, and a working electrode contact 109 located at the distal end 103 of the substrate;
The sensor electrode structure D comprises a counter electrode, which comprises a counter electrode layer 104 located at the front end 101 of the substrate, a counter electrode cable 105 located at the middle end 102 of the substrate, and a counter electrode contact 106 located at the distal end 103 of the substrate.

The sensor electrode structure D comprises a channel 114 located between the working electrode layer 107 of the working electrode and the counter electrode cable 105 of the counter electrode, and the working electrode and the counter electrode are separated by the channel 114; the width of the channel 114 is adjustable within the range of 1µm-280µm, and the length of the channel is adjustable within the range of 30µm-10,000µm.

### Embodiment 5

The structure of the sensor electrode structure E in Embodiment 5 is as shown in FIGs. 18-21.

FIG. 18 is a view of the sensor electrode structure E in Embodiment 5, where the left figure is a back view of the sensor electrode structure E and the right figure is a front view of the sensor electrode structure E. From FIG. 18, it can be seen that the sensor electrode structure E comprises a substrate 100: the substrate 100 has an L-shaped structure, and the substrate 100 comprises a front end 101, a middle end 102, and a distal end 103;
The sensor electrode structure E comprises a working electrode, which comprises a working electrode layer 107 located at the front end 101 of the substrate, a working electrode cable 108 located at the middle end 102 of the substrate, and a working electrode contact 109 located at the distal end 103 of the substrate;
The sensor electrode structure E comprises a sensing layer 115 located on the working electrode layer 107;
The sensor electrode structure E comprises a reference electrode, which comprises a reference electrode layer 113 located at the front end 101 of the substrate, a reference electrode cable 110 located at the front end 101 and the middle end 102 of the substrate, and a reference electrode contact 112 located at the distal end 103 of the substrate; the reference electrode layer 113 is provided on the reference electrode cable 110;
The sensor electrode structure E comprises a counter electrode, which comprises a counter electrode layer 104 located at the middle end 102 of the substrate, a counter electrode cable 105 located at the middle end 102 of the substrate, and a counter electrode contact 106 located at the distal end 103 of the substrate;
The working electrode is located on the back of the sensor electrode structure E, and the reference electrode and the counter electrode are located on the front of the sensor electrode structure E.

The sensor electrode structure E comprises a channel 114 located between the reference electrode cable 110 of the reference electrode and the counter electrode layer 104 of the counter electrode, and the reference electrode and the counter electrode are separated by the channel 114; the width of the channel 114 is adjustable within the range of 1µm-280µm, and the length of the channel is adjustable within the range of 30µm-10,000µm.

The sensor electrode structure E has an L-shape, which includes a first side and a second side, and an angle of 85°-95° is formed at the connection between the first side and the second side. A protruding structure 118 is formed at the outer edge of the connection between the first side and the second side.

The sensor electrode structure E comprises a protective layer 116, which is provided outside of the sensor.

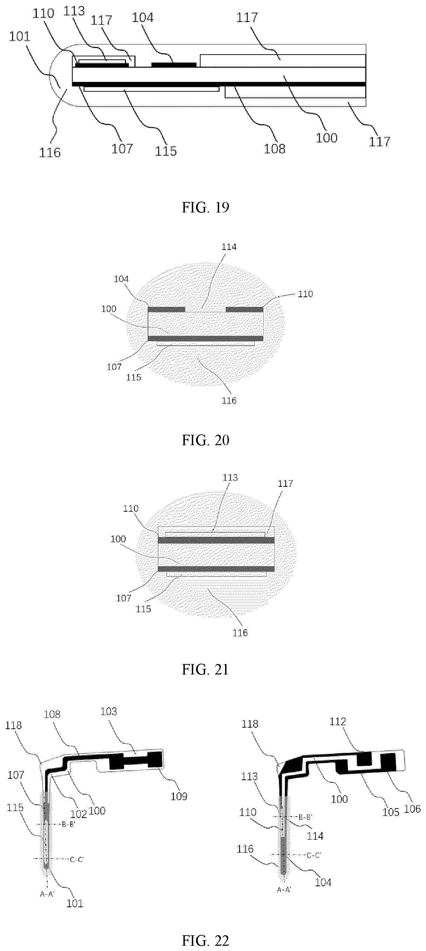

FIG. 19 is a structural diagram of the section A-A' of the sensor electrode structure E in Embodiment 5. FIG. 20 is a structural diagram of the section B-B' of the sensor electrode structure E in Embodiment 5. FIG. 21 is a structural diagram of the section C-C' of the sensor electrode structure E in Embodiment 5. It can be seen from FIGs. 19-21 that, the sensor electrode structure E also comprises a dielectric layer 117, which is provided on the reference electrode, working electrode cable, and counter electrode cable.

### Embodiment 6

The structure of the sensor electrode structure F in Embodiment 6 is as shown in FIGs. 22-25.

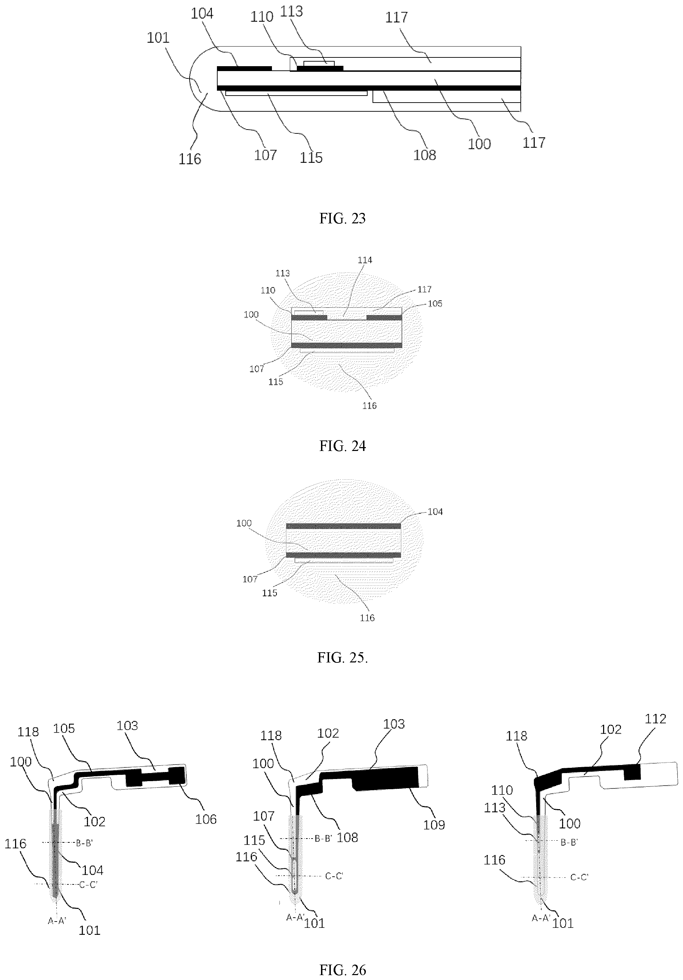

FIG. 22 is a view of the sensor electrode structure F in Embodiment 6, where the left figure is a back view of the sensor electrode structure F and the right figure is a front view of the sensor electrode structure F. FIG. 23 is a structural diagram of the section A-A' of the sensor electrode structure F in Embodiment 6. FIG. 24 is a structural diagram of the section B-B' of the sensor electrode structure F in Embodiment 6. FIG. 25 is a structural diagram of the section C-C' of the sensor electrode structure F in Embodiment 6.

The composition of the sensor electrode structure F is exactly the same as that of the sensor electrode structure E, except that the position relationship between the counter electrode and the working electrode located on the front of the sensor electrode structure has changed, that is, the sensor electrode structure F comprises a reference electrode, which comprises a reference electrode layer 113 located at the middle end 102 of the substrate, a reference electrode cable 110 located at the middle end 102 of the substrate, and a reference electrode contact 112 located at the distal end 103 of the substrate, and the reference electrode layer 113 is provided on the reference electrode cable 110;
The sensor electrode structure F comprises a counter electrode, which comprises a counter electrode layer 104 located at the front end 101 of the substrate, a counter electrode cable 105 located at the middle end 102 of the substrate, and a counter electrode contact 106 located at the distal end 103 of the substrate;
The sensor electrode structure F comprises a channel 114 located between the reference electrode layer 113 of the reference electrode and the counter electrode cable 105 of the counter electrode, and the reference electrode and the counter electrode are separated by the channel 114; the width of the channel 114 is adjustable within the range of 1µm-280µm, and the length of the channel is adjustable within the range of 30µm-10,000µm.

### Embodiment 7

The structure of the sensor electrode structure G in Embodiment 7 is as shown in FIGs. 26-29.

FIG. 26 is a view of the sensor electrode structure G in Embodiment 7, where the left figure is a back view of the sensor electrode structure G, the middle figure is a view of the middle layer of the sensor electrode structure G, and the right figure is a front view of the sensor electrode structure B. From FIG. 26, it can be seen that the sensor electrode structure G comprises a substrate 100: the substrate 100 has an L-shaped structure, and the substrate 100 comprises a front end 101, a middle end 102, and a distal end 103;
The sensor electrode structure G comprises a working electrode, which comprises a working electrode layer 107 located at the front end 101 of the substrate, a working electrode cable 108 located at the middle end 102 of the substrate, and a working electrode contact 109 located at the distal end 103 of the substrate;
The sensor electrode structure G comprises a sensing layer 115 located on the working electrode layer 107;
The sensor electrode structure G comprises a reference electrode, which comprises a reference electrode layer 113 and a reference electrode cable 110 located at the middle end 102 of the substrate, and a reference electrode contact 112 located at the distal end 103 of the substrate; the reference electrode layer 113 is provided on the reference electrode cable 110;
The sensor electrode structure G comprises a counter electrode, which comprises a counter electrode layer 104 located at the front end 101 of the substrate, a counter electrode cable 105 located at the middle end 102 of the substrate, and a counter electrode contact 106 located at the distal end 103 of the substrate;
The counter electrode is located on the back of the sensor electrode structure G, the working electrode is located in the middle of the sensor electrode structure G, and the reference electrode is located on the front of the sensor electrode structure G. That is, the working electrode and the reference electrode located at the same surface of the substrate 100 are not on the same plane.

The sensor electrode structure G comprises a channel 114 located between the working electrode cable 108 of the working electrode and the reference electrode layer 113, and the working electrode and the reference electrode are separated by the channel 114; the width of the channel 114 is adjustable within the range of 1µm-280µm, and the length of the channel is adjustable within the range of 30µm-10,000µm.

The sensor electrode structure G has an L-shape, which includes a first side and a second side, and an angle of 85°-95° is formed at the connection between the first side and the second side. A protruding structure 118 is formed at the outer edge of the connection between the first side and the second side.

The sensor electrode structure G comprises a protective layer 116, which is provided outside of the sensor.

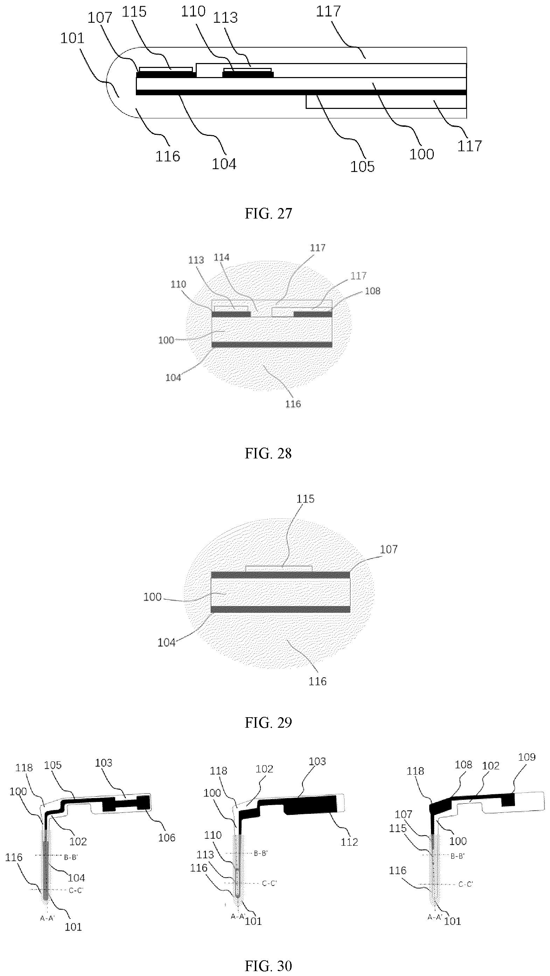

FIG. 27 is a structural diagram of the section A-A' of the sensor electrode structure G in Embodiment 7. FIG. 28 is a structural diagram of the section B-B' of the sensor electrode structure G in Embodiment 7. FIG. 29 is a structural diagram of the section C-C' of the sensor electrode structure G in Embodiment 7. It can be seen from FIGs. 27-29 that, the sensor electrode structure E also comprises a dielectric layer 117, which is provided on the reference electrode, working electrode cable, and counter electrode cable.

### Embodiment 8

The structure of the sensor electrode structure H in Embodiment 8 is as shown in FIGs. 30-33.

FIG. 30 is a view of the sensor electrode structure H in Embodiment 8, where the left figure is a back view of the sensor electrode structure H, the middle figure is a view of the middle layer of the sensor electrode structure G, and the right figure is a front view of the sensor electrode structure H. FIG. 31 is a structural diagram of the section A-A' of the sensor electrode structure H in Embodiment 8. FIG. 32 is a structural diagram of the section B-B' of the sensor electrode structure H in Embodiment 8. FIG. 33 is a structural diagram of the section C-C' of the sensor electrode structure H in Embodiment 8.

The composition of the sensor electrode structure H is exactly the same as that of the sensor electrode structure G, except that the position relationship between the reference electrode and the working electrode located on the front and in the middle layer of the sensor electrode structure has changed, that is, the counter electrode is located on the back of the sensor electrode structure G, the reference electrode is located in the middle layer of the sensor electrode structure G, and the working electrode is located on the front of the sensor electrode structure G. The working electrode and the reference electrode located at the same surface of the substrate 100 are not on the same plane.

Specifically, the sensor electrode structure H comprises a working electrode, which comprises a working electrode layer 107 and a working electrode cable 108 located at the middle end 102 of the substrate, and a working electrode contact 109 located at the distal end 103 of the substrate;
The sensor electrode structure H comprises a reference electrode, which comprises a reference electrode layer 113 located at the front end 101 of the substrate, a reference electrode cable 110 located at the front end 101 and the middle end 102 of the substrate, and a reference electrode contact 112 located at the distal end 103 of the substrate; the reference electrode layer 113 is provided on the reference electrode cable 110.

The sensor electrode structure H comprises a channel 114 located between the working electrode layer 107 of the working electrode and the reference electrode cable 110 of the reference electrode, and the working electrode and the reference electrode are separated by the channel 114; the width of the channel 114 is adjustable within the range of 1µm-280µm, and the length of the channel is adjustable within the range of 30µm-10,000µm.

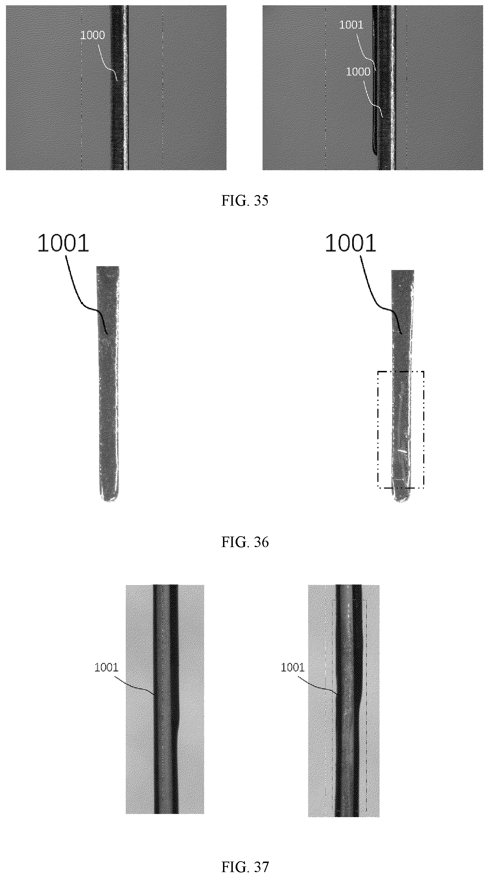

FIG. 35 is a schematic diagram of the combination of the sensor with a protruding structure and the auxiliary device (see left), as well as a schematic diagram of the combination of the sensor without a protruding structure and the auxiliary device (see right), where the reference number 1000 is the auxiliary device, and 1001 is the sensor. It can be seen from FIG. 35 that the sensor without a protruding structure is partially located outside of the auxiliary device.

FIG. 36 is a structural diagram of the sensor surface after implantation of the sensor with the protruding structure into the human body (see left), as well as a structural diagram of the sensor surface after implantation of the sensor without the protruding structure into the human body (see right). It can be seen from FIG. 36 that the sensor without a protruding structure shows damage to the sensor surface after implantation into the human body, while the sensor with a protruding structure does not show any damage to the sensor surface after implantation into the human body.

FIG. 37 is a structural diagram of the sensor after implantation of the sensor with the channel structure into the human body (see left), as well as a structural diagram of the sensor after implantation of the sensor without the channel structure (i.e., stacked structure) into the human body (see right). From FIG. 37, it can be seen that after the sensor without a channel structure (i.e. a stacked structure) is implanted into the human body, "blood stains" can be seen on the side of the sensor. The presence of "blood stains" indicates that there is significant bleeding at the wearing position when the sensor is implanted, which affects the wearing experience from the user's perspective; the sensor with a channel structure has a more suitable thickness, so there are no obvious "blood stains" on the side, indicating that the sensor with a channel structure is worn more smoothly and has a better wearing experience.

### Comparative Example 1

The other structures are as shown in Embodiment 1, with the only difference being that the width of channel 114 in the sensor of Comparative Example 1 is less than 1 µm, which can cause the working electrode cable to come into contact with the reference electrode, and the working electrode and reference electrode are prone to short circuits. The change trend chart of blood glucose after implanting the sensor into a normal human body for one day is as shown in FIG. 39. It can be seen from FIG. 39 that due to the narrow channel width in the sensor, the working electrode and the reference electrode are prone to short circuits, which can lead to high blood glucose levels. Normal blood glucose readings are generally between 3.9mM and 6.1mM.

### Comparative Example 2

The other structures are as shown in Embodiment 1, with the only difference being that the width of channel 114 in the sensor of Comparative Example 2 is greater than 280µm, which can cause the electrode/electrode cable to be too narrow, making it easy to cause an open circuit. The change trend chart of blood glucose after implanting the sensor into a normal human body for two days is as shown in FIG. 40. It can be seen from FIG. 40 that, due to the wide channel width in the sensor, the electrode/electrode cable is too narrow, which can cause data "jumping" or "jittering" during the blood glucose testing process, affecting the stability of testing data.

The above provides an explanation of the embodiments of the present application. However, the present application is not limited to the above embodiments. Any modification, equivalent replacement and improvement made within the spirit and rule of the present invention shall be incorporated in the scope of protection of the present invention.

## Claims

1. An analyte sensor, comprising a substrate, a working electrode, a reference electrode, a counter electrode, and a sensing layer; wherein the sensing layer is provided on the working electrode; wherein the working electrode, reference electrode, and counter electrode are provided at surfaces of the substrate, and two electrodes from the working electrode, reference electrode, and counter electrode located at the same surface of the substrate are spatially separated by a channel;
wherein the width of the channel is 1µm-280µm; the length of the channel is 30µm-10,000µm.

2. The analyte sensor according to claim 1, wherein the working electrode and the reference electrode are provided on a first surface of the substrate, spatially separated by a channel, and the counter electrode is provided on a second surface of the substrate opposite to the first surface; alternatively, the working electrode and the counter electrode are provided on a first surface of the substrate, spatially separated by a channel, and the reference electrode is provided on a second surface of the substrate opposite to the first surface; alternatively, the counter electrode and the reference electrode are provided on a first surface of the substrate, spatially separated by a channel, and the working electrode is provided on a second surface of the substrate opposite to the first surface; preferably, the two electrodes located at the same surface of the substrate are on the same plane; alternatively, the two electrodes located at the same surface of the substrate are partially on the same plane.

3. The analyte sensor according to claim 1 or 2, wherein the substrate comprises a front end, a middle end, and a distal end, which are sequentially connected; the substrate has an L-shape, which includes a first side and a second side, and an angle of 45°-135° is formed at the connection between the first side and the second side; the front end is located at the first side and is located at the non-connected end of the first side, the distal end is located at the second side and is located at the non-connected end of the second side, and the middle end is located at the first side and the second side, and is located at the connected end of the first side and the connected end of the second side.

4. The analyte sensor according to any of claims 1-3, wherein the working electrode comprises a working electrode layer, a working electrode cable, and a working electrode contact, and the working electrode layer is connected to the working electrode contact through the working electrode cable; the working electrode layer is located at the front end of the substrate, the working electrode cable is located at the middle end of the substrate, and the working electrode contact is located at the distal end of the substrate; alternatively, the working electrode layer and the working electrode cable are located at the middle end of the substrate, and the working electrode contact is located at the distal end of the substrate;
wherein the reference electrode comprises a reference electrode layer, a reference electrode cable, and a reference electrode contact; the reference electrode layer is provided on the reference electrode cable, and the reference electrode cable is connected to the reference electrode contact; the reference electrode layer is located at the front end of the substrate, the reference electrode cable is located at the front end and middle end of the substrate, and the reference electrode contact is located at the distal end of the substrate; alternatively, the reference electrode layer and the reference electrode cable are located at the middle end of the substrate, and the reference electrode contact is located at the distal end of the substrate;
wherein the counter electrode comprises a counter electrode layer, a counter electrode cable, and a counter electrode contact, and the counter electrode layer is connected to the counter electrode contact through the counter electrode cable; the counter electrode layer is located at the front end of the substrate, the counter electrode cable is located at the middle end of the substrate, and the counter electrode contact is located at the distal end of the substrate; alternatively, the counter electrode layer and the counter electrode cable are located at the middle end of the substrate, and the counter electrode contact is located at the distal end of the substrate;
preferably, the working electrode is configured to react with blood glucose; the reference electrode is configured to form a voltage circuit with the working electrode, providing a guarantee for converting blood glucose signals into electrochemical signals; ; the counter electrode is configured to form a current closed circuit with the working electrode, providing a guarantee for converting blood glucose signals into electrochemical signals.

5. The analyte sensor according to any of claims 1-4, wherein the working electrode comprises a working electrode layer, on which a sensing layer is provided;
preferably, the sensing layer is configured to monitor analyte, which is blood glucose.

6. The analyte sensor according to any of claims 1-5, wherein the electrode layer of one electrode and the electrode cable of the other electrode located at the same surface of the substrate are spatially separated by a channel;
preferably, the width of the channel is constant, or gradually changing.

7. The analyte sensor according to any of claims 1-6, wherein the analyte sensor has an L-shape, which includes a first side and a second side, and an angle of 45°-135° is formed at the connection between the first side and the second side;
preferably, there are two contact areas and two electrode areas on a first surface of the analyte sensor, and there is one contact area and one electrode area on a second surface of the analyte sensor opposite to the first surface; the electrode area is located at the first side of the L-shaped analyte sensor, and the contact area is located at the second side of the L-shaped analyte sensor .

8. The analyte sensor according to any of claims 1-7, wherein a protruding structure is formed at the connection between the first side and the second side; preferably, a protruding structure is formed at the outer edge of the connection between the first side and the second side.

9. The analyte sensor according to any of claims 1-8, wherein the analyte sensor further comprises a dielectric layer, which is provided on the substrate, electrode or electrode cable, and the dielectric layer is configured to provide insulation to protect the working electrode, counter electrode and reference electrode from damage;
preferably, the analyte sensor further comprises a protective layer, which is provided on the outermost layer of the sensor.

10. The analyte sensor according to any of claims 1-9, wherein the front end and partial middle end of the analyte sensor is configured to be implanted into a body, wherein a current circuit is configured to be formed using the working electrode and the counter electrode, and a voltage circuit is configured to be formed using the working electrode and the reference electrode, wherein the sensing layer located on the working electrode is configured to react with blood glucose to generate electrochemical signals;
preferably, an implant length of the analyte sensor is configured to be 1mm-20mm and the width is configured to be 0.05mm-10mm.
